Europäisches Patentamt

European Patent Office

Office européen des brevets

(11) Publication number: **0 338 730**
**A1**

## EUROPEAN PATENT APPLICATION

(21) Application number: 89303688.9

(22) Date of filing: 13.04.89

(51) Int. Cl.4: **C07C 57/03** , **C07C 51/12** , **C07C 69/52** , **C07C 67/36**

(30) Priority: 16.04.88 GB 8809027

(43) Date of publication of application:
25.10.89 Bulletin 89/43

(84) Designated Contracting States:
BE DE FR GB IT NL

(71) Applicant: **The British Petroleum Company p.l.c.**
**Britannic House Moor Lane**
**London EC2Y 9BU(GB)**

(72) Inventor: **Alper, Howard**
**University of Ottawa Department of Chemistry**
**365 Nicholas Ottawa Ontario K1N 9B4(CA)**

(74) Representative: **Barlow, Michael Thomas et al**
**BP INTERNATIONAL LIMITED Patents & Agreements Division Chertsey Road Sunbury-on-Thames Middlesex TW16 7LN(GB)**

(54) **Carbonylation of allylic alcohols.**

(57) A process for preparing an unsaturated carboxylic acid comprises contacting an allylic alcohol (e.g. allyl alcohol) with carbon monoxide and a nickel catalyst in a reaction medium comprising two immiscible phases and a phase transfer agent. One of the phases is basic and is preferably an aqueous solution of an alkali metal base.

EP 0 338 730 A1

## CARBONYLATION OF ALLYLIC ALCOHOLS

The present invention relates to a process for carbonylating allylic alcohols under phase transfer conditions using a nickel catalyst.

It is known from GB 1430415 that vinylacetic acid can be prepared by carbonylation of allyl alcohol in the presence of one or more Group VIII metals including nickel. However, the reactions described generally require quite high temperatures (e.g. 110 to 160°C) and high pressures (ca. 150 atmospheres of carbon monoxide) to achieve reasonable reaction rates. Hence a problem to be solved is to achieve high reaction rates in such a process whilst employing mild conditions in terms of temperature and pressure.

According to the present invention there is provided a process for preparing an unsaturated carboxylic acid which comprises contacting an allylic alcohol having the formula:

$CH_2 = CHC(R^1)(R^2)OH$,

wherein $R^1$ and $R^2$ are independently hydrogen or $C_1$ to $C_6$ alkyl groups, with carbon monoxide and a nickel catalyst characterised in that the reaction medium in which the process is carried out comprises two immiscible phases, one of which is basic and that a phase transfer agent is present.

The present invention solves the problem defined above by using a nickel carbonylation catalyst in conjunction with a two phase reaction medium to effect carbonylation at low temperatures and pressures than have been taught previously.

It is preferable that the two phase reaction medium consists of a first phase comprising non-polar hydrocarbon and a second phase comprising aqueous base. Whilst not wishing to be limited, it is thought that the allylic alcohol reacts with the nickel catalyst in the first phase and thereafter transfers to the second phase by means of the phase transfer agent. Once in the second phase the allylic alcohol/nickel catalyst complex reacts with carbon monoxide and base to generate a carboxylic acid salt.

It is preferable that the allylic alcohols used are those having the general formula $CH_2 = CHC(R^1)(R^2)$-OH wherein $R^1$ and $R^2$ are independently hydrogen or $C_1$ to $C_6$ alkyl groups. Using such an allylic alcohol as starting material, the carboxylic acid produced initially is believed to be one having the general formula:

$CH_2 = CHC(R^1)(R^2)COOH$

However, it will be appreciated that under the reaction conditions, this initial product may undergo double bond isomerisation to yield isomers of greater thermodynamic stability than the initial product. An example of such would be the isomerisation of the initial product to produce an unsaturated carboxylic acid which is conjugated. It will also be appreciated that such subsequent isomerisations are probably kinetically controlled and indeed it is possible that isomerisation actually takes place in some cases before carbonylation. The above should not therefore be regarded as a description of exactly what takes place in the reaction mechanistically, but more of a rationalisation on theoretical grounds of the product observed.

More preferably, the allylic alcohol is one in which $R^1$ and $R^2$ are independently selected from hydrogen, methyl and ethyl. The most preferred starting material is allyl alcohol ($R^1 = R^2 = H$).

Whilst any nickel salt can be used in principle as a source of the catalyst, a preferred salt is the cyanide which it is thought generates the reactive anion $(CO)_3NiCN^-$ under the reaction conditions.

The first phase, i.e. the non-polar hydrocarbon, is preferably one which is high boiling, e.g. benzene, toluene, xylene, decane etc. As regards the second phase, it is convenient to use an aqueous solution of an inorganic base (e.g. an alkali metal base such as NaOH, KOH, etc.) for reasons of cost. The second phase is preferably strongly basic, i.e. preferably greater than 1M in base.

The phase transfer agent is preferably a quaternary ammonium halide having the formula $R_4NX$ where X is halide and the $R_4$ groups are independently $C_1$ to $C_{30}$ alkyl with the proviso that at least one $R_4$ group is $C_8$ to $C_{30}$ alkyl. Most preferred are species where the other $R_4$ groups are methyl. For example, it has been found that cetyltrimethylammonium halides are particularly good with the bromide salt being preferred.

The reaction is preferably carried out at a temperature in the range 60 to 120°C and at a pressure of up to 50 atmospheres. Typical allylic alcohol to nickel catalyst molar ratios are in the range 10:1 to 100:1.

It is a feature of the above process that high yields of the carboxylic acid can be achieved with an atmospheric pressure of carbon monoxide. Higher pressures can be used to increase yields further.

The process of the present invention is suitably carried out by feeding the various components to an appropriate reaction vessel, the contents of which are continuously agitated. After reaction, the components are removed from the vessel and the two phases separated. The product can be removed from the phase containing base by acidification.

The process is now illustrated by the following Examples.

Experimental Method

A mixture of aqueous sodium hydroxide (20ml - see Table for concentration), nickel cyanide tetrahydrate (1mmol), cetyltrimethylammonium bromide (58mg) and toluene (20ml) was stirred at 90 ± 5°C until the aqueous phase turned red (ca 0.5 - 2.5hr). 10mmol of the appropriate allylic alcohol (see Table) and para-xylene (0.1mmol internal standard) were added over a period of three hours. During this time the mixture was kept under an atmosphere of carbon monoxide. The yield of carboxylic acid was measured by gas chromatography (after work up as outlined below) at the reaction times indicated in Table.

At the end of the reaction, the layers were separated and crushed ice added to the aqueous phase. The cooled aqueous layer was then acidified with 10% hydrochloric acid and extracted with ether. The ether was dried over sodium sulphate and concentrated by a rotary evaporator to cause precipitation of the carboxylic acid. Conversions and yields are given in the Table for Examples 1 to 5 which employ five different allylic alcohols as feed.

Table

| Example | Allylic Alcohol | Sodium Hydroxide Conc. (M) | Time hr. | Product | Conversion % | Isolated Yield |
|---|---|---|---|---|---|---|
| 1 | $CH_2 = CHCH_2OH$ | 5 | 20 | $CH_3CH = CHCOOH$ | 90 | 64 |
| 2 | $CH_2 = CHC(CH_3)_2OH$ | 5 | 6.5 | $(CH_3)_2C = CHCH_2COOH$ | 90 | 40 |
| | | 7.5 | 20 | $(CH_3)_2C = CHCH_2COOH$ | 90 | 53 |
| 3 | $CH_2 = C(CH_3)CH_2OH$ | 5 | 20 | $(CH_3)_2C = CHCOOH$ | 95 | 50 |
| | | | | $(CH_2) = C(CH_3)CH_2COOH$ | | 4 |
| | | 1.1 | 20 | $(CH_3)_2C = CHCOOH$ | 70 | 32 |
| | | | | $CH_2 = (CH_3)CH_2COOH$ | | 23 |
| | | 2.5 | 16 | $(CH_3)_2C = CHCOOH$ | 46 | 15 |
| | | | | $CH_2 = C(CH_3)CH_2COOH$ | | 5 |
| 4 | $CH_2 = CHCH(OH)C_2H_5$ | 7.5 | 5 | $C_3H_7CH = CHCOOH$ | 84 | 20 |
| | | | | $C_2H_5CH = CHCH_2COOH$ | | 58 |
| 5 | $CH_2 = CHC(OH)(CH_3)C_2H_5$ | 1.25 | 17 | $(C_2H_5)(CH_3)C = CHCH_2COOH$ | 50 | 4 |
| | | | | $(C_2H_5)(CH_3)CHCH = CHCOOH$ | | 36 |
| | | 4.5 | 22 | $(C_2H_5)(CH_3)C = CHCH_2COOH$ | 76 | 38 |
| | | | | $(C_2H_5)(CH_3)CHCH = CHCOOH$ | | 8 |

EP 0 338 730 A1

## Claims

1. A process for preparing an unsaturated carboxylic acid which comprises contacting an allylic alcohol having the formula:

$$CH_2 = CHC(R^1)(R^2)OH$$

wherein $R^1$ and $R^2$ are independently hydrogen or $C_1$ to $C_6$ alkyl with carbon monoxide and a nickel catalyst characterised in that the reaction medium in which the process is carried out comprises two immiscible phases, one of which is basic and that a phase transfer agent is present.

2. A process as claimed in claim 1 characterised in that the allylic alcohol is one having the formula defined above wherein $R^1$ and $R^2$ are independently hydrogen, methyl or ethyl.

3. A process as claimed in claim 2 characterised in that the allylic alcohol is allyl alcohol.

4. A process as claimed in claim 1 characterised in that the phase transfer agent is a quaternary ammonium halide of formula $R_4NX$ where X is halide, three of the $R_4$ groups are methyl and the fourth $R_4$ group is a $C_8$ to $C_{30}$ alkyl group.

5. A process as claimed in claim 1 wherein the first phase is a high boiling, non-polar hydrocarbon and the second phase is an aqueous solution of an alkali metal base.

6. A process as claimed in claim 1 characterised in that the nickel catalyst source is a cyanide salt.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | US-A-4 189 608 (KURKOV) <br> * Claim 1 * <br> ----- | 1 | C 07 C 57/03 <br> C 07 C 51/12 <br> C 07 C 69/52 <br> C 07 C 67/36 |
| | | | **TECHNICAL FIELDS SEARCHED (Int. Cl.4)** <br><br> C 07 C 57/00 <br> C 07 C 51/00 <br> C 07 C 67/00 <br> C 07 C 69/00 |

The present search report has been drawn up for all claims

| Place of search | Date of completion of the search | Examiner |
|---|---|---|
| THE HAGUE | 07-07-1989 | KLAG M.J. |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons
 
& : member of the same patent family, corresponding
document

EPO FORM 1503 03.82 (P0401)